# EUROPEAN PATENT APPLICATION

(11) **EP 3 501 502 A1**
(43) Date of publication of application: **26.06.2019**
(21) Application number: 17208852.8
(22) Date of filing: 20.12.2017
(51) Int. Cl.: A61K 9/20, A61K 9/24, A61K 31/40, A61K 31/4422

(54) **FIXED DOSED PHARMACEUTICAL COMPOSITIONS COMPRISING AMLODIPINE, RAMIPRIL AND ATORVASTATIN**

(71) Applicant: Midas Pharma GmbH, 55218 Ingelheim (DE)
(72) Inventor: KAHM, Andreas, D-55218 Ingelheim (DE); JÜNEMANN, Daniel, D-65346 Eltville (DE)
(74) Representative: Drescher, Christian

(57) **Abstract**

The present invention relates to fixed dosed immediate release pharmaceutical compositions comprising the three active substances Amlodipine, Atorvastatin and Ramipril, or their pharmaceutically acceptable salts or esters, for the treatment of hypertension and related diseases.

## Description

### BACKGROUND OF THE INVENTION

The present invention relates to fixed dosed pharmaceutical compositions comprising the three active pharmaceutical ingredients (APIs) Amlodipine, Atorvastatin and Ramipril, or their pharmaceutically acceptable salts or esters, with improved stability for the treatment of hypertension and related diseases.

Amlodipine is the international nonproprietary name (INN) of (*RS*)-3-ethyl-5-methyl-2-[(2-aminoethoxy)methyl]-4-(2-chlorophenyl)-6-methyl-1,4-dihydropyridine-3,5-dicarboxylate with the following chemical structure:

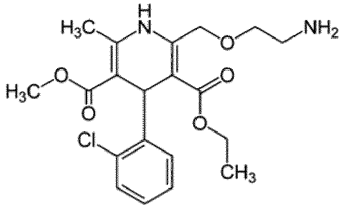

Pharmaceutical compositions of the calcium channel blocker (CCB) Amlodipine in the modification of its benzene sulfonate ("besilate") salt are registered and marketed under the trade name NORVASC® for the treatment of, inter alia, hypertension.

Atorvastatin is the INN of (3*R*,5*R*)-7-[2-(4-fluoro-phenyl)-3-phenyl-4-(phenylcarbamoyl)-5-propan-2-ylpyrrol-1-yl]-3,5-dihydroxyheptanoic acid with the following chemical structure:

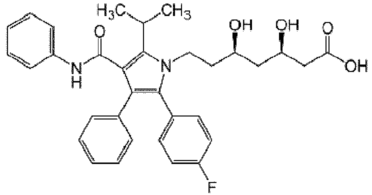

Pharmaceutical compositions of the 3-hydroxy-3-methyl-glutaryl-coenzyme A (HMG-CoA) reductase inhibitor Atorvastatin are registered and marketed under the trade names SORTIS® and LIPITOR® for the treatment of hypercholesterolemia and the prevention of cardiovascular diseases.

Ramipril is the INN of (2S,3aS,6aS)-1-[(2S)-2-[[(2S)-1-ethoxy-1-oxo-4-phenylbutan-2-yl]amino]-propanoyl]-3,3a,4,5,6,6a-hexahydro-2H-cyclopenta-[b]pyrrole-2-carboxylic acid which has the following chemical structure:

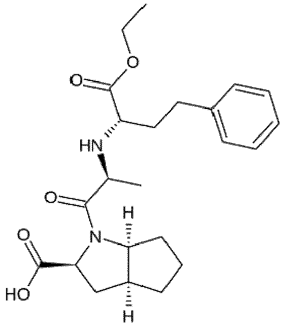

Pharmaceutical compositions of the Angiotensin converting enzyme (ACE) inhibitor Ramipril are registered and marketed under the trade name DELIX® for the treatment of, inter alia, cardiovascular disorders and hypertension.

Several fixed dose combinations comprising one or two of these three APIs already exist. Fixed-dosed pharmaceutical compositions comprising Amlodipine and Atorvastatin are currently registered and marketed under the trade name CADUET®. Fixed-dosed pharmaceutical compositions comprising Amlodipine and Ramipril are registered and marketed under the trade name TONOTEC®.

Fixed-dosed pharmaceutical compositions comprising Atorvastatin, Ramipril and Acetylsalicylic acid are registered and marketed under the trade name TRINOMIA®. However, TRINOMIA® is not a uniform pharmaceutical dosage form, but a capsule which contains, depending on the strengths, three or even more film-coated immediate release tablets.

As regards fixed-dosed pharmaceutical combinations comprising Amlodipine, Ramipril and Atorvastatin, no such fixed-dosed pharmaceutical combination has been registered or marketed yet.

It is desirable to include multiple active ingredients into a single pharmaceutical composition for various reasons, e.g. to provide a single medication to a patient rather than multiple medications. In other words, the inherent advantages of fixed-dose combination therapies result in improved patient compliance because fewer medications have to be consumed by the patient.

The qualitative compositions (excluding coating materials) and strengths of the mono-products NORVASC®, DELIX®, SORTIS® as well as the combinational products CADUET® and TONOTEC® are displayed in Table 1.

**Table 1**

| **Atorvastatin** | **SORTIS®** | **NOHVASC®** | **DELIX®** | **CADUET®** | **TONETEC®** |
|---|---|---|---|---|---|
| Active pharmaceutical Ingredients (APIs) | Atorvastatin | Amlodipine | Ramipril | Amlodipine + | Amlodipine + Ramipril |
| Dosage form | Film-coated tablet | Tablet | Tablet | Film-coated tablet | Capsule |
| Strengths [mg] | 2.5, 5, 10 | 1.25, 2.5, 5, 10 | 10, 20, 40, 80 | 2.5/10, 2.5/20, 2.5/40, 5/10, 5/20, 5/40, 5/80, 10/10, 10/20, 10/40, 10/80 | 5/5, 5/10, 10/5, 10/10 |
| Calcium carbonate | Yes | - | - | Yes | - |
| Microcrystalline cellulose | Yes | Yes | Yes | Yes | Yes |
| Pregelatinized starch | - | - | Yes | Yes | - |
| Lactose monohydrate | Yes | - | - | - | - |
| Hydroxypropyl cellulose | Yes | - | - | Yes | - |
| Polysorbate 80 | Yes | - | - | Yes | - |
| Calcium hydrogen phosphate | - | Yes | - | - | - |
| Hypromellose | - | - | Yes | - | Yes |
| Croscarmellose sodium | Yes | - | - | Yes | - |
| Sodium starch glycolate | - | Yes | - | - | - |
| Crospovidone | - | - | - | - | Yes |
| Glyceryl dibehenate | - | - | - | - | Yes |
| Colloidal sillicon dioxide | - | - | - | Yes | - |
| Magnesium stearate | Yes | Yes | - | Yes | - |
| Sodium stearyl fumarate | - | - | Yes | - | - |

From table 1 it can be concluded that the excipients and the compositions of the mono-products as well as the combinational products are quite different from each other. Accordingly, neither the compositions of the marketed mono-compounds nor the compositions of any combinational product can give a clear guidance how to formulate a stable and fixe-dosed oral pharmaceutical composition comprising Amlodipine, Ramipril and Atorvastatin which is bioequivalent to NORVASC®, DELIX®, and SORTIS®.

During the development of fixed-dosed combinational products comprising two or even more active ingredients, multiple challenges have to be overcome. For example, one active ingredient may be incompatible with another API or the stability of one active ingredient may be affected due to its incompatibility with an excipient that is commonly used in order to stabilize another API.

In the present case, Amlodipine is known to be incompatible with the excipient lactose, rendering the composition of SORTIS® unsuitable to serve as a basis for a fixed dose combination of Atorvastatin and Amlodipine.

Amlodipine besilate, being weakly basic, is highly hygroscopic and sensitive to hydrolysis and the exposure to light. It exhibits physical properties as low bulk densities, poor flow or uniformity of dosage units. It is also susceptible to static electricity and highly cohesive. Amlodipine besilate is slightly soluble in water and has an absolute bioavailability of 64-90%. The formation of its main degradant is enhanced at low pH-values.

Ramipril, on the other hand, is weakly acidic in nature and its main degradant ramipril-diketopiperazine ("DKP", also referred to as "impurity D") is formed at high pH-values. Besides exhibiting certain undesirable flow characteristics such as stickiness, it is very sensitive to degradation under oxidative conditions and wherein moisture is present. In addition, the formation of DKP is increased by mechanical stress during the formulation of Ramipril.

CN 101612403 discloses medicinal combinations of calcium channel blockers, ACE inhibitors and HMG-CoA reductase inhibitor and a pharmaceutically acceptable carrier. Specifically, orally disintegrating tablets (ODT) comprising Levamlodipine besilate, Ramipril and Atorvastatin are disclosed. According to the US-FDA, ODT formulations should be considered as solid oral preparations that disintegrate rapidly in the oral cavity, with an in-vitro disintegration time of approximately 30 seconds or less, when based on the United States Pharmacopeia (USP) disintegration test method or alternative. In a definition of the European Pharmacopoeia, ODTs disintegrate within 3min in water.

In summary, it can be concluded that there is no guidance in the prior art how to design, develop and manufacture a uniform and stable fixed dosed combination of Amlodipine, Atorvastatin and Ramipril which is bioequivalent to NORVASC®, DELIX®, SORTIS® and how to deal with all the obstacles described above.

It has now been found that fixed dose pharmaceutical formulations of Amlodipine, Atorvastatin and Ramipril according to the present invention satisfy all requirements as regards the stability and the processability of all three active substances as well as their dissolution profile compared to their respective brand products.

As can be seen in table 2, up to 48 different fixed-dose combinations with different strengths of Amlodipine, Ramipril and are theoretically possible.

**Table 2**

| **Fixed dose combination** | **Amlodipine** | **Ramipril** | **Atorvastatin** |
|---|---|---|---|
| 1. | | 1.25 mg | 10 mg |
| 2. | | | 20 mg |
| 3. | | | 40 mg |
| 4. | | | 80 mg |
| 5. | | 2.5 mg | 10 mg |
| 6. | | | 20 mg |
| 7. | | | 40 mg |
| 8. | 2.5 mg | | 80 mg |
| 9. | | 5 mg | 10 mg |
| 10. | | | 20 mg |
| 11. | | | 40 mg |
| 12. | | | 80 mg |
| 13. | | 10 mg | 10 mg |
| 14. | | | 20 mg |
| 15. | | | 40 mg |
| 16. | | | 80 mg |
| 17. | | 1.25 mg | 10 mg |
| 18. | | | 20 mg |
| 19. | | | 40 mg |
| 20. | | | 80 mg |
| 21. | | 2.5 mg | 10 mg |
| 22. | | | 20 mg |
| 23. | | | 40 mg |
| 24. | | | 80 mg |
| 25. | 5 mg | 5 mg | 10 mg |
| 26. | | | 20 mg |
| 27. | | | 40 mg |
| 28. | | | 80 mg |
| 29. | | 10 mg | 10 mg |
| 30. | | | 20 mg |
| 31. | | | 40 mg |
| 32. | | | 80 mg |
| 33. | | 1.25 mg | 10 mg |
| 34. | | | 20 mg |
| 35. | | | 40 mg |
| 36. | | | 80 mg |
| 37. | | 2.5 mg | 10 mg |
| 38. | | | 20 mg |
| 39. | | | 40 mg |
| 40. | 10 mg | | 80 mg |
| 41. | | 5 mg | 10 mg |
| 42. | | | 20 mg |
| 43. | | | 40 mg |
| 44. | | | 80 mg |
| 45. | | 10 mg | 10 mg |
| 46. | | | 20 mg |
| 47. | | | 40 mg |
| 48. | | | 80 mg |

For several technical, regulatory and commercial reasons it is economically not attractive to develop, register and manufacture 48 different fixed-dose combinations of Amlodipine, Atorvastatin and Ramipril. It has now been found how to focus on those fixed-dose combinations which allow a cost efficient registration process as well as economical manufacturing.

### BRIEF DESCRIPTION OF THE FIGURES

Figure 1 shows the dissolution of formulations of 10mg Amlodipine, 10mg Ramipril and 40mg Atorvastatin prepared according to example 1 compared to DELIX® and NORVASC® and SORTIS®.
Figure 2 shows the dissolution of formulations of 5mg Amlodipine, 10mg Ramipril and 20mg Atorvastatin prepared according to example 1 compared to DELIX® and NORVASC® and SORTIS®.
Figure 3 shows the dissolution of formulations of 10mg Amlodipine, 10mg Ramipril and 40mg Atorvastatin prepared according to example 2 compared to DELIX® and NORVASC® and SORTIS®.

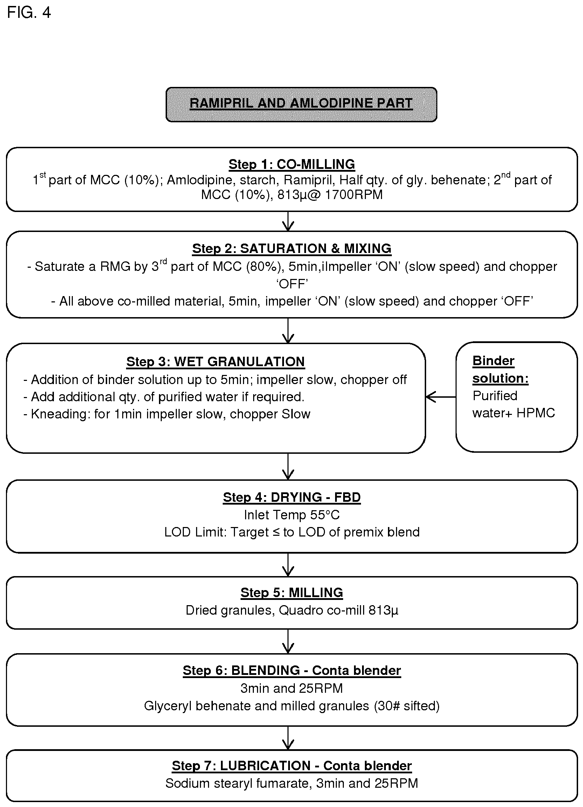

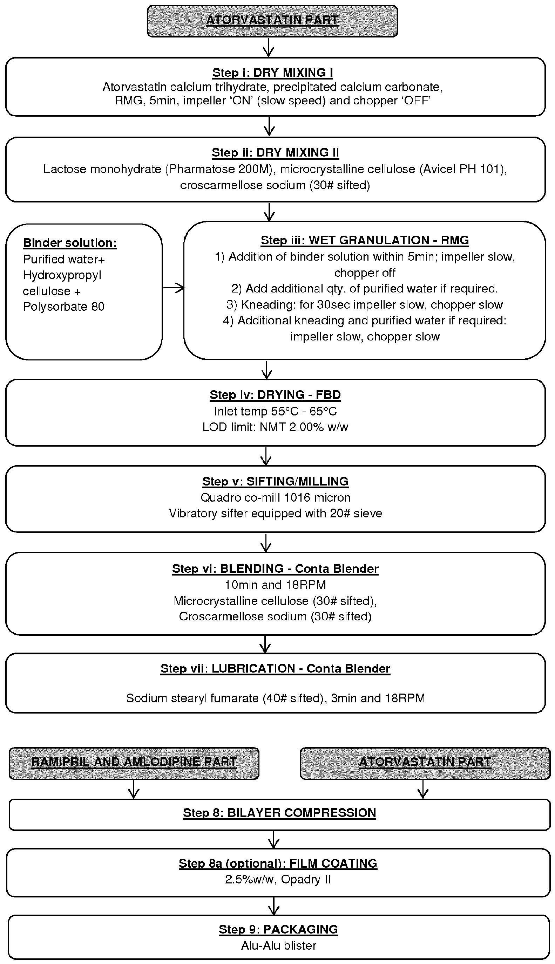

Figure 4 shows a flow chart of the manufacturing process of formulations according to example 1.

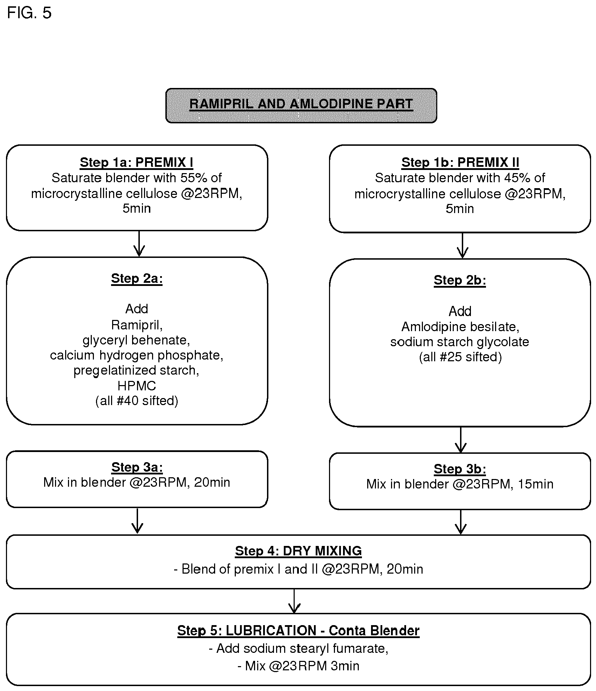

Figure 5 shows a flow chart of the manufacturing process of formulations according to example 2.

### DETAILED DESCRIPTION OF THE INVENTION

The present invention relates to fixed dosed immediate release pharmaceutical compositions comprising the active substances Amlodipine, Ramipril and Atorvastatin, or their pharmaceutically acceptable salts or esters.

The immediate release pharmaceutical compositions according to the present invention are characterized by the fact that they disintegrate in not less than 3 minutes, preferably within from 4 to 10 minutes under standard conditions as set in 2.9.1 of PharmEur (version 9.3).

In preferred embodiments of the present invention, the fixed dosed immediate release pharmaceutical compositions are in form of a capsule or a tablet, preferably a bilayer tablet.

In preferred embodiments of the present invention, Amlodipine is utilized in the form of its benzenesulfonate (besilate) salt and Atorvastatin is utilized in the form of its calcium salt.

Amlodipine besilate may be manufactured according to processes known in the art, e.g. as disclosed in patent application EP 0 244 944 A2. Ramipril may be manufactured according to processes known in the art, e.g. as disclosed in patent application EP 0 079 022 A2. A process for the preparation of Atorvastatin calcium is disclosed in WO 1997/003959 A1.

The pharmaceutical compositions of the present invention may comprise excipients such as filler(s), binder(s), lubricant(s), stabilizer(s), solubilizer(s), disintegrant(s), glidant(s) etc.

In the context of the present invention, the terms filler(s), binder(s), lubricant(s), stabilizer(s), solubilizer(s), disintegrant(s), glidant(s) etc. shall be understood as including a single compound but also multiple compounds.

The pharmaceutical compositions of the present invention comprise one or more pharmaceutically acceptable filler(s). Pharmaceutically acceptable fillers according to the present invention are microcrystalline cellulose (MCC), calcium phosphates, calcium carbonate, magnesium carbonate, starch, powder cellulose, calcium silicate, sorbitol, dextrin, kaolin, magnesium oxide, calcium sulfate, xylitol, lactose and mixtures thereof. The total amount of filler(s) is in the range from 30wt.-% to 90wt.-%, based on the total weight of the composition. Preferred filler(s) are microcrystalline cellulose, calcium hydrogen phosphate and mixtures thereof. Lactose may be used in pharmaceutical compositions in form of a bilayer tablet wherein lactose is not comprised in the Amlodipine containing layer.

The pharmaceutical compositions of the present invention comprise one or more pharmaceutically acceptable binder(s). Pharmaceutically acceptable binders according to the present invention are hydroxypropyl methylcellulose (HPMC), hydroxypropyl cellulose (HPC), dihydroxypropyl cellulose, methyl cellulose, hydroxyethyl cellulose, ethyl cellulose, sodium carboxymethyl cellulose, polyethylene glycol, polymethacrylates, sodium alginate, polyvinylpyrrolidone, pregelatinized starch, vinylpyrrolidone/vinylacetate copolymers (copovidone) and mixtures thereof. The total amount of binder(s) is in the range from 0.5wt.-% to 4%wt.-%, preferably in the range of from 1wt.-% to 3%wt.-%, based on the total weight of the composition. Preferred binders are hydroxypropyl methylcellulose, and hydroxypropyl cellulose.

The pharmaceutical compositions of the present invention comprise one or more pharmaceutically acceptable disintegrant(s). Suitable disintegrant(s) according to the present invention are carboxymethyl cellulose, crospovidone, sodium starch glycolate, croscarmellose, sodium carboxymethyl starch and mixtures thereof. The total amount of disintegrant(s) is in the range from 0.25wt.-% to 4wt.-%, preferably in the range from 0.5wt.-% to 2wt.-%, based on the total weight of the composition. Preferred disintegrant(s) are croscarmellose sodium, sodium starch glycolate and mixtures thereof.

The pharmaceutical compositions of the present invention comprise one or more pharmaceutically acceptable lubricant(s). Suitable lubricants according to the present invention are sodium stearyl fumarate, magnesium stearate, calcium stearate, sodium stearate, stearic acid, hexanedioic acid, hydrogenated vegetable oil, glycerol fumarate and mixtures thereof. The total amount of lubricant(s) is in the range from 0.25wt.-% to 2wt.-%, preferably in the range from 0.5wt.-% to 1wt.-%, based on the total weight of the composition. The preferred lubricant is sodium stearyl fumarate.

Certain pharmaceutical compositions of the present invention may comprise a pharmaceutically acceptable stabilizer for Ramipril. The ratio of Ramipril and the Ramipril stabilizer is in the range from 1:0.5 to 1:2 by weight, preferably in the range from 1:1 to 1:1.5 by weight. The preferred stabilizer for Ramipril is glyceryl behenate.

Certain pharmaceutical compositions of the present invention may comprise a pharmaceutically acceptable stabilizer for Atorvastatin. The ratio of Atorvastatin and the Atorvastatin stabilizer is in the range from 1:1 to 1:5 by weight, preferably in the range from 1:2 to 1:4 by weight. The preferred stabilizer for Atorvastatin is calcium carbonate.

Certain pharmaceutical compositions of the present invention may comprise a pharmaceutically acceptable solubilizer for Atorvastatin. The ratio of Atorvastatin and the Atorvastatin solubilizer is in the range from 5:1 to 40:1 by weight, preferably in the range from 10:1 to 25:1 by weight. The preferred solubilizer is polysorbate 80.

The pharmaceutical compositions according to the present invention may optionally comprise pharmaceutically acceptable glidant(s) such as colloidal silicon dioxide, talcum, magnesium carbonate and mixtures thereof. The amount of glidant(s) is preferably in the range from 0.1wt.-% to 1.5wt.-%, relative to the total weight of the composition.

The present invention relates to fixed dosed immediate release pharmaceutical compositions comprising the active substances Amlodipine, Ramipril, Atorvastatin, or their pharmaceutically acceptable salts or esters, and one or more pharmaceutically acceptable binder(s), one or more pharmaceutically acceptable filler(s), one or more pharmaceutically acceptable stabilizer(s), one or more pharmaceutically acceptable disintegrant(s), one or more pharmaceutically acceptable lubricant(s) and optionally one or more pharmaceutically acceptable solubilizer(s).

Preferably, the present invention relates to fixed dosed immediate release pharmaceutical compositions comprising the active substances Amlodipine besilate, Ramipril, Atorvastatin calcium, from 0.5wt.-% to 4%wt.-% of one or more pharmaceutically acceptable binder(s), from 30wt.-% to 90 wt.-% one or more pharmaceutically acceptable filler(s), one or more pharmaceutically acceptable stabilizer(s), from 0.25wt.-% to 4wt.-% of one or more pharmaceutically acceptable disintegrant(s), from 0.25wt.-% to 2wt.-% of one or more pharmaceutically acceptable lubricant(s) and optionally one or more pharmaceutically acceptable solubilizer(s), all wt.-% being based on the total weight of the composition.

In a preferred embodiment of the invention, the fixed dosed immediate release pharmaceutical compositions further comprise calcium carbonate as a stabilizer for Atorvastatin wherein the ratio of Atorvastatin and calcium carbonate is in the range from 1:1 to 1:5 by weight, and glyceryl behenate as a stabilizer for Ramipril wherein the ratio of Ramipril and glyceryl behenate is in the range from 1:0.5 to 1:2 by weight.

As will be shown below, the fixed dosed pharmaceutical compositions of the present invention exhibit excellent stability. In particular, the content of impurity D of Ramipril after 3 months storage at 40°C/75%RH is less than 5.0% w/w, preferably less than 3.50% w/w.

As will be shown below, the fixed dosed pharmaceutical compositions of the present invention also exhibit very similar dissolution profiles compared to their respective brand products as regards all three active substances.

The fixed dosed immediate release pharmaceutical compositions according to the present invention may be used in the treatment of hypertension and related diseases.

As already indicated above, it is economically not attractive to develop, register and manufacture all of the 48 theoretically possible fixed dosed compositions of Amlodipine, Ramipril and Atorvastatin.

It has been found that certain sets of fixed dosed pharmaceutical compositions containing Amlodipine, Ramipril and Atorvastatin are advantageous from a regulatory and economic point of view.

As will be explained below, this is true for certain pharmaceutical compositions in the form of bilayer tablets comprising 5mg/5mg or 5mg/10mg or 10mg/10mg of Ramipril/Amlodipine in a first layer and 10mg or 20mg or 40mg of Atorvastatin in a second layer.

From a regulatory standpoint, two different layers may, in principal, be considered independent from each other.

If the total weight of one layer is 285mg or more, the relative amount of Amlodipine besilate in the 10mg strength remains below 5wt.-% compared to the total weight of the layer. Provided this, there is no need to perform additional bioequivalence studies for a lower strength, e.g. the 5mg strength, if Amlodipine besilate is solely replaced by a filler so that the total weight of the layer remains constant.

However, the total weight of the layer should not exceed 345mg because then the 5mg strength would contain less than 2wt.-% Amlodipine besilate compared to the total weight of the layer. Accordingly, the product would be considered as a "non-standard" product, resulting in the need of costly and thus undesirable re-validation if the batch size is changed.

For the same reason, compositions with only 2.5mg of Amlodipine are not preferred embodiments of the present invention.

As regards Ramipril, the minimum weight of the layer of 285mg as set out above results in a Ramipril content of 3.5wt.-% compared to the total weight of the layer for the 10mg strength.

The same rationale can be applied to achieve dosage forms with 5mg Amlodipine, resulting in a total weight of the Amlodipine and Ramipril containing layer of from 142.5mg to 172.5mg. In this layer containing 5mg Amlodipine, Ramipril may be present in amounts of 5mg or 10mg.

If prepared in the way described above, the different strengths of Amlodipine besilate and Ramipril are "dose-proportional".

The combination of 5mg of Ramipril with 10mg of Amlodipine does, however, not meet the regulatory demands explained above. For the same reason, compositions with only 2.5mg or 1.25mg of Ramipril do also not meet the regulatory demands.

As regards the Atorvastatin containing layer, three different strengths (10mg, 20mg and 40mg) may be manufactured from a single pre-mix, i.e. only the total amount of the pre-mix is adapted between 100mg (for the 10mg strength), 200mg (for the 20mg strength) or 400mg (for the 40mg strength), i.e. the different strengths are weight-proportional.

In addition, this "single pre-mix" approach renders the manufacturing process much more cost efficient. However, this approach does not suit for compositions comprising 80mg Atorvastatin since the corresponding Atorvastatin layer with a total weight of 800mg would result in tablets that are too heavy and too big.

The combination of a "dose-proportional" approach for the Amlodipine besilate and Ramipril containing layer and a "weight-proportional" approach for the Atorvastatin containing layer is advantageous from a regulatory perspective because, for the reasons set out above, up to nine different combinations of strengths may be registered with only one bioequivalence study. In addition, all nine different combinations of strengths may be produced from only three pre-mixes (two pre-mixes of Amlodipine/Ramipril and one pre-mix of Atorvastatin), which renders both the product development and the manufacturing very cost efficient. Accordingly, a preferred embodiment of the present invention relates to a set of fixed dosed pharmaceutical compositions in the form of bilayer tablets, comprising 5mg/5mg, 5mg/10mg or 10mg/10mg of Amlodipine/Ramipril and 10mg, 20mg or 40mg of Atorvastatin. In particular, it relates to 5mg/5mg/10mg, 5mg/5mg/20mg, 5mg/5mg/40mg, 5mg/10mg/10mg, 5mg/10mg/20mg, 5mg/10mg/40mg, 10mg/10mg/10mg, 10mg/10mg/20mg and 10mg/10mg/40mg of Amlodipine/Ramipril/Atorvastatin. An overview of the preferred set of strengths is provided in table 3.

**Table 3**

| | ***5*/*5*/*10*** | ***5*/*5*/*20*** | **5/5/40** | ***5*/*10*/*10*** | ***5*/*10*/*20*** | ***5*/*10*/*40*** | **10/10/10** | ***10*/*10*/*20*** | ***10*/*10*/*40*** |
|---|---|---|---|---|---|---|---|---|---|
| | | | | | | | | | |
| **Amlodipine** | 5mg | | | | | | 10mg | | |
| **Amlodipine besilate in layer** | 4.9% | | | 2.45% | | | 4.9% | | |
| | 5mg | | | 10mg | | | 10mg | | |
| **Ramipril % in layer** | 3.5% | | | | | | | | |
| **∑ Ramipril + Amlodipine layer** | 142.50mg | | | 285.00mg | | | | | |
| | | | | | | | | | |
| **Atorvastatin** | 10mg | 20mg | 40mg | 10mg | 20mg | 40mg | 10mg | 20mg | 40mg |
| **Atorvastatin calcium in layer** | 10.85% | | | | | | | | |
| **∑ Atovarstatin layer** | 100mg | 200mg | 400mg | 1100mg | 200mg | 400mg | 1100mg | 200mg | 400mg |
| | | | | | | | | | |
| **Total weignt of the composition** | **242.50mg** | **342.50mg** | **542.50mg** | **385mg** | **485mg** | **585mg** | **385mg** | **485mg** | **685mg** |

For reasons of patient compliance, the actives should be combined in a way so that either all actives are incorporated in relatively low strengths or all actives are incorporated in relatively high strengths. In particular, the ratio of Amlodipine to Atorvastatin and Ramipril to Atorvastatin shall remain in the range from 1:2 to 1:4. Accordingly, a particular preferred set of fixed dosed pharmaceutical compositions according to the present invention comprise 5mg/5mg/10mg, 5mg/5mg/20mg, 5mg/10mg/20mg, 10mg/10mg/20mg and 10mg/10mg/40mg of Amlodipine/Ramipril/Atorvastatin. An overview of the preferred strengths is provided in table 4.

**Table 4**

| | **Amiodipine/Ramipril/Atorvastatin** | | | | |
|---|---|---|---|---|---|
| | **5/5/10mg** | **5/5/20mg** | **5/10/10mg** | **10/10/20mg** | **10/10/40mg** |
| **Amiodipine besilate** | 5mg | | | 10mg | |
| | 4.9% | | 2.45% | 4.9% | |
| **Ramipril** | 5mg | | 10mg | | |
| | 3.5% | | | | |
| **Ramipril + Amlodipine part** | 142.50mg | | 285.00mg | | |
| **Atorvastatin** | 10mg | 20mg | | | 40mg |
| **Atorvastatin calcium %** | 10.85% | | | | |
| **Total weight Atorvastatin part** | 100mg | 200mg | | | 400mg |
| **Total weight of composition** | **242.50mg** | **342.50mg** | **485.00mg** | | **685.00mg** |

Accordingly, the present invention also relates to fixed dosed immediate release pharmaceutical compositions in form of bilayer tablets for the treatment of hypertension, wherein the first layer comprises 5mg or 10mg Ramipril, 5mg or 10mg Amlodipine as Amlodipine besilate and one or more pharmaceutically acceptable binder(s), one or more pharmaceutically acceptable filler(s), one or more pharmaceutically acceptable stabilizer(s) and one or more pharmaceutically acceptable lubricant(s), and the second layer comprises 10mg, 20mg or 40mg Atorvastatin as Atorvastatin calcium, one or more pharmaceutically acceptable binder(s), one or more pharmaceutically acceptable filler(s), one or more pharmaceutically acceptable stabilizer(s), one or more pharmaceutically acceptable disintegrant(s), one or more pharmaceutically acceptable lubricant(s).

The invention also relates to processes for the manufacture of uniform, stable pharmaceutical compositions comprising Amlodipine, Ramipril and Atorvastatin, or their pharmaceutically acceptable salts or esters.

The pharmaceutical compositions according to the present invention may be manufactured by a process comprising the steps of:
a) preparing a first mixture comprising Ramipril and Amlodipine besilate and a first set of excipients by means of wet granulation or dry mixing;
b) preparing Atorvastatin granules with a second set of excipients,
c) combining the mixture of step a) and the granules of step b), optionally with a third set of excipients; and
d) filling the product of step c) into capsules or compressing it into tablets, preferably into bilayer tablets.

Detailed exemplary processes for the manufacture of pharmaceutical compositions according to the present invention are provided in the examples below.

### EXAMPLES

### Example 1 - Pharmaceutical compositions

### a) Preparation of the Ramipril and Amlodipine layer by wet granulation

A first part (10%) of microcrystalline cellulose is passed through 0.8mm sieve of Quadro comill at 1700RPM, followed by a premix of Amlodipine besilate and Ramipril with 50% of the pregelatinized starch and 50% of the glyceryl behenate. The mill is then rinsed by passing a second part (10%) of microcrystalline cellulose (step 1).

A high sheer mixer is saturated with the remaining third part (80%) of microcrystalline cellulose and the combined materials of step 1 are added and mixed (step 2).

The binder solution containing hydroxypropyl methyl cellulose is added and granules are formed (step 3).

The granules are then dried in a fluid bed dryer (step 4) and milled (step 5).

The dried granules of Ramipril + Amlodipine are then charged into a conta blender, the remaining half quantity of glyceryl behenate is added and mixed (step 6). The mixture is finally lubricated with sodium stearyl fumarate (step 7).

The respective quantities are displayed in table 5.

**Table 5**

| **Strengths** | | **10/10/10/40mg** | | **10/10/20mg** | | **10/5/20mg** | | **5/5/20mg** | | **5/5/10mg** | |
|---|---|---|---|---|---|---|---|---|---|---|---|
| **RAMIPRIL AND AMLODIPINE Layer** | | | | | | | | | | | |
| **Intragranular part** | | | | | | | | | | | |
| **Sr. no** | **Ingredients** | **mg/tab** | **%w/w*** | **mg/tab** | **%w/w*** | **mg/tab** | **%w/w*** | **mg/tab** | **%w/w*** | **mg/tab** | **%w/w*** |
| 1 | Ramipril | 10.00 | 3.51 | 10.00 | 3.51 | 10.00 | 3.51 | 5.00 | 3.51 | 5.00 | 3.51 |
| 2 | Amlodipine besilate | 13.88 | 4.87 | 13.88 | 4.87 | 6.94 | 2.44 | 6.94 | 4.87 | 6.94 | 4.87 |
| 3 | Microcrystalline cellulose | 187.00 | 65.61 | 187.00 | 65.61 | 193.94 | 68.05 | 93.50 | 65.61 | 93.50 | 65.61 |
| 4 | Pregelatinized starch | 57.00 | 20.00 | 57.00 | 20.00 | 57.00 | 20.00 | 28.50 | 20.00 | 28.50 | 20.00 |
| 5 | Glyceryl behenate | 5.70 | 2.00 | 5.70 | 2.00 | 5.70 | 2.00 | 2.85 | 2.00 | 2.85 | 2.00 |
| **Total** | | **273.58** | **95.99** | **273.58** | **95.99** | **273.58** | **95.99** | **136.79** | **95.99** | **136.79** | **95.99** |

| **Binder solution** | | | | | | | | | | | |
|---|---|---|---|---|---|---|---|---|---|---|---|
| 6 | HPMC | 4.27 | 1.50 | 4.27 | 1.50 | 4.27 | 1.50 | 2.135 | 1.50 | 2.135 | 1.50 |
| 7 | Purified water | qs | NA | qs | NA | qs | NA | qs | NA | qs | NA |
| **Total** | | **277.85** | **97.49** | **277.85** | **97.49** | **277.85** | **97.49** | **138.93** | **97.49** | **138.93** | **97.49** |

| **Extragranular part** | | | | | | | | | | | |
|---|---|---|---|---|---|---|---|---|---|---|---|
| 9 | Glyceryl behenate | 5.70 | 2.00 | 5.70 | 2.00 | 5.70 | 2.00 | 2.85 | 2.00 | 2.85 | 2.00 |
| 10 | Sodium stearyl fumarate | 1.45 | 0.51 | 1.45 | 0.51 | 1.45 | 0.51 | 0.725 | 0.51 | 0.725 | 0.51 |
| **Total Ramipril and Amlodipine layer** | | **285.00** | **100.00** | **285.00** | **100.00** | **285.00** | **100.00** | **142.50** | **100.00** | **142.50** | **100.00** |

| | | | | | | | | | | | |
|---|---|---|---|---|---|---|---|---|---|---|---|
| * All w/w amounts are wt.-%, based on the weight of the total composition | | | | | | | | | | | |

### b) Preparation of the Atorvastatin layer

Atorvastatin calcium is co-sifted with calcium carbonate and dry-mixed (step i).

Lactose monohydrate, microcrystalline cellulose and croscarmellose sodium are added and dry-mixed (step ii).

A binder solution prepared by addition of dispensed polysorbate 80 into a solution of hydroxypropyl cellulose and purified water is added to the mixture obtained in step ii and wet granulated (step iii).

The granules are then dried in a fluid bed dryer (step iv), sifted and milled (step v).

The dried Atorvastatin granules are then blended with microcrystalline cellulose and croscarmellose sodium (step vi) and finally lubricated with sodium stearyl fumarate (step vii).

The respective quantities are displayed in table 6.

**Table 6**

| **Strengths** | | **10/10/40mg** | | **10/10/20mg** | | **10/10/20mg** | | **5/5/20mg** | | **5/5/20mg** | |
|---|---|---|---|---|---|---|---|---|---|---|---|
| **ATORVASTATIN Layer** | | | | | | | | | | | |
| **Intragranular part** | | | | | | | | | | | |
| **Sr. no** | **Ingredients** | **mg/tab** | **%w/w*** | **mg/tab** | **%w/w*** | **mg/tab** | **%w/w*** | **mg/tab** | **%w/w*** | **mg/tab** | **%w/w*** |
| 1 | Atorvastatin calcium trihydrate | 43.40 | 10.85 | 21.70 | 10.85 | 21.70 | 10.85 | 21.70 | 10.85 | 10.85 | 10.85 |
| 2 | Microcrystalline cellulose | 45.00 | 11.25 | 22.50 | 11.25 | 22.50 | 11.25 | 22.50 | 11.25 | 11.25 | 11.25 |
| 3 | Croscarmellose sodium | 16.00 | 4.00 | 8.00 | 4.00 | 8.00 | 4.00 | 8.00 | 4.00 | 4.00 | 4.00 |
| 4 | Lactose monohydrate | 103.55 | 25.89 | 51.78 | 25.89 | 51.78 | 25.89 | 51.78 | 25.89 | 25.89 | 25.89 |
| 5 | Calcium carbonate | 130.00 | 32.50 | 65.00 | 32.50 | 65.00 | 32.50 | 65.00 | 32.50 | 32.50 | 32.50 |
| **Total** | | **337.95** | **84.49** | **168.98** | **84.49** | **168.98** | **84.49** | **168.98** | **84.49** | **84.49** | **84.49** |

| **Binder solution** | | | | | | | | | | | |
|---|---|---|---|---|---|---|---|---|---|---|---|
| 6 | Hydroxypropyl cellulose | 12.00 | 3.00 | 6.00 | 3.00 | 6.00 | 3.00 | 6.00 | 3.00 | 3.00 | 3.00 |
| 7 | Polysorbate 80 | 2.00 | 0.50 | 1.00 | 0.50 | 1.00 | 0.50 | 1.00 | 0.50 | 0.50 | 0.50 |
| 8 | Purified water | qs | NA | qs | NA | qs | NA | qs | NA | qs | NA |
| **Total** | | **351.95** | **87.99** | **175.98** | **87.99** | **175.98** | **87.99** | **175.98** | **87.99** | **87.99** | **87.99** |

| **Extra granular part** | | | | | | | | | | | |
|---|---|---|---|---|---|---|---|---|---|---|---|
| 9 | Microcrystalline cellulose | 38.05 | 9.51 | 19.03 | 9.51 | 19.03 | 9.51 | 19.03 | 9.51 | 9.51 | 9.51 |
| 10 | Croscarmellose sodium | 8.00 | 2.00 | 4.00 | 2.00 | 4.00 | 2.00 | 4.00 | 2.00 | 2.00 | 2.00 |
| 11 | Sodium stearyl fumarate | 2.00 | 0.50 | 1.00 | 0.50 | 1.00 | 0.50 | 1.00 | 0.50 | 0.50 | 0.50 |
| **Total Atorvastatin layer** | | **400.00** | **100** | **200.00** | **100** | **200.00** | **100** | **200.00** | **100** | **100.00** | **100** |

| | | | | | | | | | | | |
|---|---|---|---|---|---|---|---|---|---|---|---|
| * All w/w amounts are wt.-%, based on the weight of the total composition | | | | | | | | | | | |

### c) Preparation of the bilayer tablet

The Ramipril-Amlodipine part and the Atorvastatin part are compressed into a bilayer tablet (step 8), optionally film coated (step 8a) and packed into Alu-Alu blisters (step 9).

The respective quantities are displayed in table 7.

**Table 7**

| **Strengths** | | **10/10/40mg** | | **0/10/20mg** | | **10/5/20mg** | | **5/5/20mg** | | **5/5/10mg** | |
|---|---|---|---|---|---|---|---|---|---|---|---|
| **Bilayer tablet** | | | | | | | | | | | |
| **Sr. no** | **Layer** | **mg/tab** | **%w/w*** | **mg/tab** | **%w/w*** | **mg/tab** | **%w/w*** | **mg/tab** | **%w/w*** | **mg/tab** | **%w/w*** |
| 12 | Ramipril and Amlodipine layer | 285.0 | 41.61 | 285.0 | 58.76 | 285.0 | 58.76 | 142.5 | 41.61 | 142.5 | 58.76 |
| 13 | Atorvastatin layer | 400.0 | 58.39 | 200.0 | 41.24 | 200.0 | 41.24 | 200.0 | 58.39 | 100.0 | 41.24 |
| | **Total weight of tablet** | **685.0** | **100** | **485.0** | **100** | **485.0** | **100** | **342.5** | **100** | **242.5** | **100** |

| | | | | | | | | | | | |
|---|---|---|---|---|---|---|---|---|---|---|---|
| * All w/w amounts are wt.-%, based on the weight of the total composition | | | | | | | | | | | |

An exemplary batch of tablets containing 10mg Amlodipine, 10mg Ramipril and 40mg Atorvastatin was submitted to a stability study under accelerated conditions (40°C, 75% rel. humidity). As can be seen in table 8, the compositions according to example 1 exhibit excellent stability. In particular, Ramipril impurity D remains far below its limit.

**Table 8**

| **Impurity related active** | **RRT** | **Limits** | **Initial** | **1 Month (40°C/75% RH)** | **2 Months (40°C/75% RH)** | **3 Months (40°C/75% RH** |
|---|---|---|---|---|---|---|
| **Ramipril** | **Ramiprilate** | **6.0**¹⁾ | ND | 0.06 | 0.14 | 0.20 |
| | **Impurity D** | | 0.14 | 1.30 | 2.03 | 2.70 |
| | **RRT 0.193** | **0.5**²⁾ | 0.14 | 0.14 | 0.17 | 0.13 |
| | **RRT 1.255** | **0.5**²⁾ | 0.13 | 0.13 | 0.13 | 0.13 |
| **Amlodiprine** | **Amlodiprine adduct** | **0.2**³⁾ | BQL | 0.11 | 0.13 | 0.13 |
| | **Amlodiprine Imp D** | **0.3**³⁾ | 0.06 | 0.09 | 0.11 | 0.13 |
| | **RRT 1.103** | **0.5**²⁾ | 0.06 | 0.08 | ND | ND |
| | **RRT 1.450** | **0.5**²⁾ | 0.11 | 0.05 | 0.07 | 0.07 |
| | **RRT 1.461** | **0.5**²⁾ | ND | 0.05 | ND | ND |
| **Atorvastatin** | **Impurity-H** | **2.0**³⁾ | 0.07 | 0.19 | 0.22 | 0.26 |
| | **Impurity-D1** | **0.35**³⁾ | ND | 0.06 | 0.05 | 0.06 |
| | **Impurity-HD2** | | N | 0.07 | 0.08 | 0.11 |
| **Total impurities** | | NA | 0.71 | 2.33 | 3.13 | 3.92 |

| | | | | | | |
|---|---|---|---|---|---|---|
| ¹⁾BP (Ramipril tabs); ²⁾ ICH Q 3 B; ³⁾ USP (Atorvastatin + Amlodipine tabs) | | | | | | |

Exemplary batches of tablets containing 10mg Amlodipine, 10mg Ramipril and 40mg Atorvastatin and 5mg Amlodipine, 10mg Ramipril and 20mg Atorvastatin are submitted to dissolution studies under standard conditions as displayed in table 9.

**Table 9**

| **Drug** | **Reference** | **Apparatus** | **Speed [RPM]** | **Medui** | **Volume [ml]** |
|---|---|---|---|---|---|
| Amlodipine besilate, Atorvastatin, Ramipril | USP | Basket | 100 | Acetate buffer (pH 4.5) | 900 |

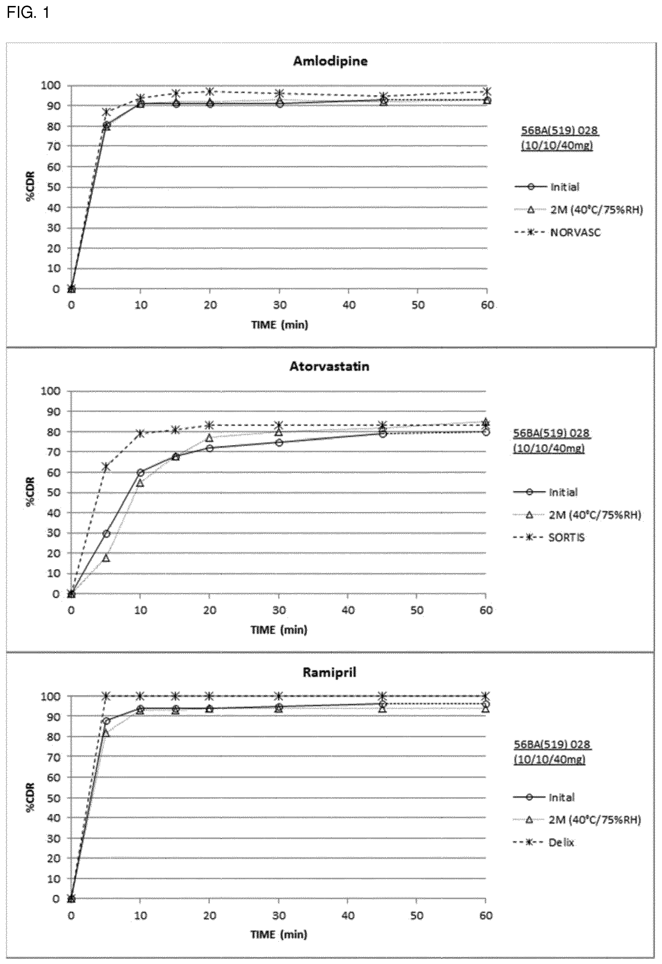

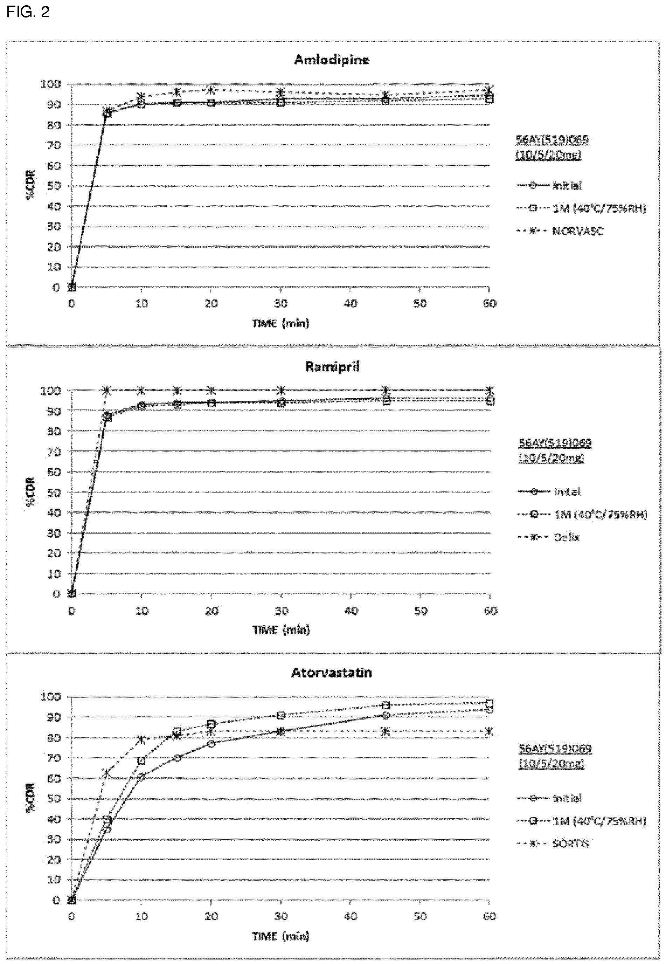

Tables 10 and 11, figs. 1 and 2 indicate that the in-vitro-dissolution profiles for all actives are not only similar to the respective branded product, but also do not change significantly after having been submitted to stability testing.

**Table 10**

| **Active** | **Ramipril 10mg** | | | **Amlodipine 10mg** | | | **Atorvastatine 40 mg** | | |
|---|---|---|---|---|---|---|---|---|---|
| **Time Points** | **Initial** | **1M 40/75** | **2M 40/75** | **Initial** | **1M 40/75** | **2M 40/75** | **Initial** | **1M 40/75** | **2M 40/75** |
| **0min** | 0 | 0 | 0 | 0 | 0 | 0 | 0 | 0 | 0 |
| **5min** | 88 | 72 | 82 | 81 | 65 | 80 | 30 | 11 | 18 |
| **10min** | 94 | 92 | 93 | 91 | 85 | 91 | 60 | 35 | 55 |
| **15min** | 94 | 93 | 93 | 91 | 87 | 92 | 68 | 47 | 68 |
| **20min** | ND | 93 | 94 | ND | 89 | 92 | ND | 58 | 77 |
| **30min** | 95 | 94 | 94 | 91 | 88 | 93 | 75 | 65 | 80 |
| **45min** | 96 | 94 | 94 | 93 | 88 | 92 | 79 | 74 | 82 |
| **60min** | 96 | 95 | 94 | 93 | 89 | 93 | 80 | 85 | 85 |

**Table 11**

| **Active** | **Ramipril 10mg** | | **Amlodipine 5mg** | | **Atorvastatin 20mg** | |
|---|---|---|---|---|---|---|
| **Time Points** | **Initial** | **1M 40/75** | **Initial** | **1M 40/75** | **Initial** | **1M 40/75** |
| **0min** | 0 | 0 | 0 | 0 | 0 | 0 |
| **5min** | 88 | 87 | 86 | 86 | 35 | 40 |
| **10min** | 93 | 92 | 90 | 90 | 61 | 69 |
| **15min** | 94 | 93 | 91 | 91 | 70 | 83 |
| **20min** | 94 | 94 | 91 | 91 | 77 | 87 |
| **30min** | 95 | 94 | 93 | 91 | 83 | |
| **45min** | 96 | 95 | 93 | 92 | 91 | 96 |
| **60min** | 96 | 95 | 95 | 93 | 94 | |

In table 12, additional data such as disintegration time, hardness and friability of an exemplary batch of tablets are displayed.

**Table 12**

| **Parameters** | **Bilayer Tablet (10/5/20mg) (285mg + 200mg) = 485 mg** |
|---|---|
| **Hardness [N]** | 142 to 150 |
| **Thickness [mm]** | 4.46 to 4.52 |
| **Disintegration time (DT)* [min:sec]** | Initial: 04:40 |
| | 1 Month (40°C/75%RH): 04:40 |
| **Friability @ 300 RPM [%]** | 0.28 |

| | |
|---|---|
| * PharmEur 2.9.1 (version 9.3) | |

### Example 2 - Alternative pharmaceutical compositions

### a) Preparation of the Ramipril and Amlodipine layer by dry mixing:

A first premix (premix I) is prepared by saturating a blender with a first part of the microcrystalline cellulose (55% w/w) (step 1a), followed by addition of Ramipril, glyceryl behenate, calcium hydrogen phosphate, pregelatinized starch and HPMC (step 2a) and mixing (step 3a).

A second premix (premix II) is prepared by saturating a blender with a second part of the microcrystalline cellulose (45% w/w) (step 1b), followed by addition of Amlodipine besilate and sodium starch glycolate (step 2b) and mixing (step 3b).

Premix I and premix II are then blended and dry mixed (step 4a). Finally, the blend is submitted to lubrication (step 5a).

The respective quantities are displayed in table 13.

**Table 13**

| **Strengths** | | **10/10/40mg** | | **10/10/20mg** | | **10/5/20mg** | | **5/5/20mg** | | **5/5/10mg** | |
|---|---|---|---|---|---|---|---|---|---|---|---|
| **RAMIPRIL AND AMLODIPINE Layer** | | | | | | | | | | | |
| **Premix I** | | | | | | | | | | | |
| **Sr. no** | **Ingredients** | **mg/tab** | **%w/w** | **mg/tab** | **%w/w** | **mg/tab** | **%w/w** | **mg/tab** | **%w/w** | **mg/tab** | **%w/w** |
| 1 | Ramipril | 10.00 | 3.5 | 10.00 | 3.5 | 10.00 | 3.5 | 5.00 | 3.5 | 5.000 | 3.5 |
| 2 | Microcrystalline cellulose | 92.00 | 32.3 | 92.00 | 32.3 | 92.00 | 32.3 | 46.00 | 32.3 | 46.00 | 32.3 |
| 3 | Glyceryl behenate | 12.00 | 4.2 | 12.00 | 4.2 | 12.00 | 4.2 | 6.00 | 4.2 | 6.00 | 4.2 |
| 4 | Calcium hydrogen phosphate | 44.00 | 15.4 | 44.00 | 15.4 | 44.00 | 15.4 | 22.00 | 15.4 | 22.00 | 15.4 |
| 5 | Pregelatinized starch | 28.00 | 9.8 | 28.00 | 9.8 | 28.00 | 9.8 | 14.00 | 9.8 | 14.00 | 9.8 |
| 6 | HPMC | 3.00 | 1.1 | 3.00 | 1.1 | 3.00 | 1.1 | 1.50 | 1.1 | 1.50 | 1.1 |
| **Total** | | **189.00** | **66.3** | **189.00** | **66.3** | **189.00** | **66.3** | **94.50** | **66.3** | **94.50** | **66.3** |

| **Premix II** | | | | | | | | | | | |
|---|---|---|---|---|---|---|---|---|---|---|---|
| 7 | Amlodipine besilate | 13.88 | 4,8 | 13.88 | 4.8 | 6.94 | 2.4 | 6.94 | 4.8 | 6.94 | 4.8 |
| 8 | Microcrystalline cellulose | 74.30 | 26.1 | 74.30 | 26.1 | 81.24 | 28.5 | 37.15 | 26.1 | 37.15 | 26.1 |
| 9 | Sodium starch glycolate | 5.00 | 1.8 | 5.00 | 1.8 | 5.00 | 1.8 | 2.50 | 1.8 | 2.50 | 1.8 |
| **Total** | | **282.18** | **99.0** | **282.18** | **99.0** | **282.18** | **99.0** | **141.09** | **99.0** | **141.09** | **99.0** |

| **Lubrication** | | | | | | | | | | | |
|---|---|---|---|---|---|---|---|---|---|---|---|
| 10 | Sodium stearyl fumarate | 2.82 | 1.0 | 2.82 | 1.0 | 2.82 | 1.0 | 1.41 | 1.0 | 1.41 | 1.0 |
| **Total Ramipril and Amlodipine layer** | | **285.00** | **100** | **285.00** | **100** | **285.00** | **100** | **142.50** | **100** | **142.50** | **100** |

The Atorvastatin layer is prepared as described in example 1 b.

The bilayer tablet is prepared as described in example 1c.

An exemplary batch of tablets containing 10mg Amlodipine, 10mg Ramipril and 40mg Atorvastatin was submitted to a stability study under accelerated conditions (40°C, 75% rel. humidity). As can be seen in table 14, the compositions according to example 2 exhibit excellent stability. In particular, Ramipril impurity D (DKP) remains far below its limit.

**Table 14**

| **Impurity related to active** | **RRT** | **Limits** | **Initial** | **1 Month (40°C/75% RH)** | **2 Months (40°C/75%RH)** |
|---|---|---|---|---|---|
| **Ramipril** | **Ramiprilate** | 6.0 ¹⁾ | 0.07 | 0.65 | 0.83 |
| | **Impurity D** | | 0.17 | 1.50 | 3.09 |
| | **RRT 0.938** | 0.5 ²⁾ | ND | BQL | 0.05 |
| | **RRT 1.302** | 0.5 ²⁾ | ND | ND | 0.06 |
| | **RRT 1.414** | 0.5 ²⁾ | ND | BQL | 0.08 |
| **Amlodipine** | **Amlodipine adduct** | 0.2 ³⁾ | ND | 0.07 | 0.09 |
| | **Amlodipine imp D** | 0.3 ³⁾ | 0.05 | 0.14 | 0.17 |
| | **RRT 1.438** | 0.5 ²⁾ | 0.06 | ND | 0.06 |
| **Atorvastatin** | **Impurity-H** | 2.0 ²⁾ | 0.10 | 0.14 | 0.20 |
| | **Impurity-D1** | 0.35 ²⁾ | ND | 0.05 | 0.07 |
| | **Impurity-D2** | | 0.05 | ND | 0.05 |
| **Total impurities** | | NA | 0.50 | 2.55 | 4.75 |

| | | | | | |
|---|---|---|---|---|---|
| ¹⁾BP (Ramipril tabs); ²⁾ ICH Q 3 B; ³⁾ USP (Atorvastatin + Amlodipine tabs) | | | | | |

An exemplary batch of tablets containing 10mg Amlodipine, 10mg Ramipril and 40mg Atorvastatin (356BA(519)061) is submitted to dissolution studies under standard conditions as displayed in table 10.

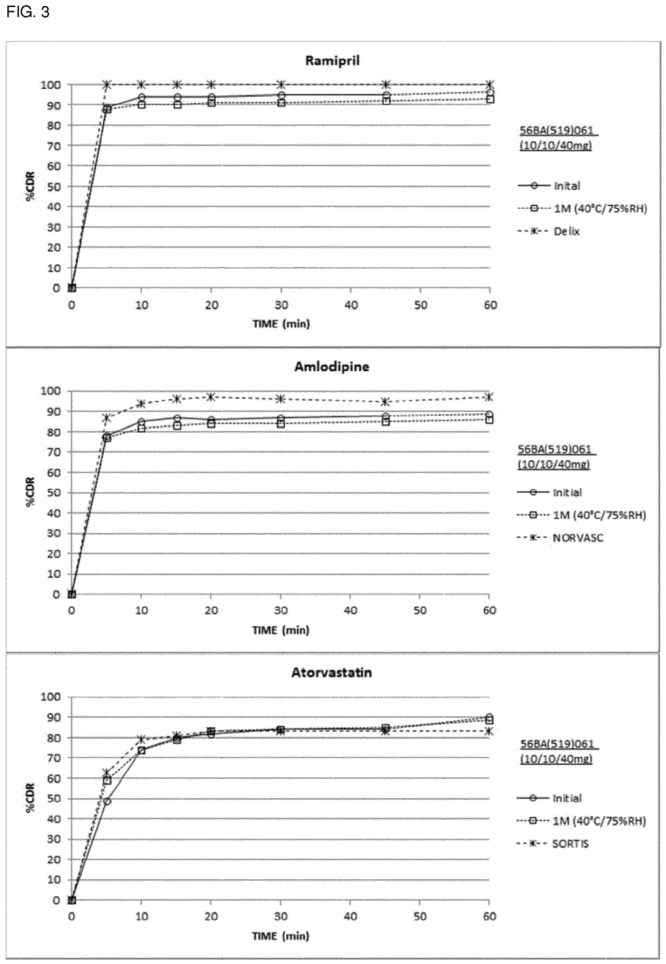

Table 15 and fig. 3 indicate that the in-vitro-dissolution profiles for all actives are not only similar to the respective branded product, but also do not change significantly after having been submitted to stability testing.

**Table 15**

| **Active** | **Ramipril** | | **Amlodipine** | | **Atorvastatin** | |
|---|---|---|---|---|---|---|
| **Time Points** | **Initial** | **1M 40/75** | **Initial** | **1M 40/75** | **Initial** | **1M 40/75** |
| **0** | 0 | 0 | 0 | 0 | 0 | 0 |
| **5** | 89 | 88 | 78 | 77 | 49 | 59 |
| **10** | 94 | 90 | 85 | 82 | 74 | 74 |
| **15** | 94 | 90 | 87 | 83 | 80 | 79 |
| **20** | 94 | 91 | 86 | 84 | 82 | 83 |
| **30** | 95 | 91 | 87 | 84 | 84 | 84 |
| **45** | 95 | 92 | 88 | 85 | 84 | 85 |
| 60 | 96 | 93 | 89 | 86 | 90 | 89 |

## Claims

1. A fixed dosed immediate release pharmaceutical composition comprising the active substances Amlodipine, Ramipril and Atorvastatin, or their pharmaceutically acceptable salts or esters.

2. The fixed dosed immediate release pharmaceutical composition according to claim 1 in form of a capsule or a tablet, preferably a bilayer tablet.

3. The fixed dosed pharmaceutical composition according to claim 1 or claim 2, further comprising one or more pharmaceutically acceptable binder(s), one or more pharmaceutically acceptable filler(s), one or more pharmaceutically acceptable stabilizer(s), one or more pharmaceutically acceptable disintegrant(s), one or more pharmaceutically acceptable lubricant(s), and optionally one or more pharmaceutically acceptable solubilizer(s).

4. The fixed dosed pharmaceutical composition according to any of the preceding claims comprising from 30wt.-% to 90wt.-%, based on the total weight of the composition, of one or more pharmaceutically acceptable filler(s).

5. The fixed dosed pharmaceutical composition according to any of the preceding claims comprising from 0.5wt.-% to 4wt.-%, preferably in the range of from 1wt.-% to 3wt.-%, based on the total weight of the composition, of one or more pharmaceutically acceptable binder(s).

6. The fixed dosed pharmaceutical composition according to any of the preceding claims comprising from 0.25wt.-% to 4wt.-%, preferably from 0.5wt.-% to 2wt.-%, based on the total weight of the composition, of one or more pharmaceutically acceptable disintegrant(s).

7. The fixed dosed pharmaceutical composition according to any of the preceding claims comprising from 0.25wt.-% to 2wt.-%, preferably from 0.5wt.-% to 1wt.-%, based on the total weight of the composition, of one or more pharmaceutically acceptable lubricant(s).

8. The fixed dosed pharmaceutical composition according any of the preceding claims, comprising glyceryl behenate as a stabilizer for Ramipril in a Ramipril:glyceryl behenate-ratio of from 1:0.5 to 1:2 by weight.

9. The fixed dosed pharmaceutical composition according to any of the preceding claims comprising calcium carbonate as a stabilizer for Atorvastatin in an Atorvastatin:calcium carbonate-ratio of from 1:1 to 1:5 by weight.

10. A fixed dosed pharmaceutical composition according to any of the preceding claims, **characterized in that** the content of impurity D of Ramipril after 3 months storage at 40°C/75%RH is less than 5.0% w/w, preferably less than 3.50% w/w.

11. A fixed dosed pharmaceutical composition according any of the preceding claims **characterized in that** it disintegrates in not less than 3 minutes, preferably within from 4 to 10 minutes under standard conditions as set in 2.9.1 of PharmEur (version 9.3).

12. A set of fixed dosed pharmaceutical compositions according to any of the preceding claims in the form of bilayer tablets, comprising 5mg/5mg or 5mg/10mg or 10mg/10mg of Ramipril/Amlodipine in a first layer and 10mg or 20mg or 40mg of Atorvastatin in a second layer.

13. A set of fixed dosed pharmaceutical compositions according to claim 10, comprising 5mg/5mg/10mg, 5mg/5mg/20mg, 5mg/5mg/40mg, 5mg/10mg/10mg, 5mg/10mg/20mg, 5mg/10mg/40mg, 10mg/10mg/10mg, 10mg/10mg/20mg and 10mg/10mg/40mg of Amlodipine/Ramipril/ Atorvastatin.

14. A set of fixed dosed pharmaceutical compositions according to claim 11, comprising 5mg/5mg/10mg, 5mg/5mg/20mg, 5mg/10mg/20mg, 10mg/10mg/20mg and 10mg/10mg/40mg of Amlodipine/Ramipril/Atorvastatin for the treatment of hypertension.

15. A process for the manufacture of a pharmaceutical composition according to any of the preceding claims, comprising the steps of:
a) preparing a mixture comprising Ramipril and Amlodipine besilate and a first set of excipients, comprising one or more filler(s), one or more binder(s) and one or more stabilizer(s) by either means of of wet granulation or by means of dry mixing,
b) preparing Atorvastatin granules with a second set of excipients, comprising one or more filler(s) and one or more lubricant(s);
c) combining the mixture of step a) and the granules of step b), optionally with a third set of excipients;
d) filling the product of step c) into capsules or compressing it into tablets, preferably into bilayer tablets.
